# EUROPEAN PATENT APPLICATION

(11) **EP 3 967 776 A1**
(43) Date of publication of application: **16.03.2022**
(21) Application number: 20795112.0
(22) Date of filing: 21.04.2020
(51) Int. Cl.: C12Q 1/6883, G01N 33/569

(54) **USE OF P44 AS MARKER FOR DIAGNOSING ANAPLASMOSIS**

(30) Priority: 26.04.2019 KR 20190049225
(71) Applicant: Industry-Academic Cooperation Foundation, Chosun University, Gwangju 61452 (KR)
(72) Inventor: KIM, Dong Min, Gwangju 61704 (KR)
(74) Representative: Murgitroyd & Company
(86) International application number: PCT/KR2020/005252
(87) International publication number: WO 2020/218802

(57) **Abstract**

A novel use of P₄₄ as a marker for predicting or diagnosing anaplasmosis is disclosed. A diagnostic composition for anaplasmosis containing a P₄₄ gene, a primer set or probe for detecting *Anaplasma phagocytophilum,* a kit for diagnosing anaplasmosis, and a method for providing information to diagnose infection with *Anaplasma phagocytophilum* are also disclosed. P₄₄, which is a novel biomarker for diagnosing anaplasmosis, is a multi-copy gene and exists in a large number of copies in the *Anaplasma phagocytophilum* genome, thus having the effect of detecting *Anaplasma phagocytophilum* infection at high sensitivity using only a small amount of DNA compared to conventional diagnostic marker genes. In addition, the primer set or probe for detecting and amplifying P₄₄ is capable of providing rapid and easy detection of anaplasmosis with high specificity and sensitivity, making it appropriate for early detection of anaplasmosis.

## Description

### BACKGROUND OF THE INVENTION

### Field of the Invention

The present invention relates to a novel use of P₄₄ as a marker for predicting or diagnosing anaplasmosis.

### Description of the Related Art

Anaplasmosis is an acute febrile illness that is transmitted through the bite of a tick carrying *Anaplasma phagocytophilum* and exhibits a nonspecific acute febrile clinical pattern. Young adults with anaplasmosis exhibit mild symptoms, whereas elderly or immunocompromised anaplasmosis patients exhibit thrombocytopenia, leukopenia, elevated levels of gamma glutamyltransferase, and other more severe symptoms. Anaplasmosis responds well to antibiotic treatment, but some patients may die due to complications if diagnosis is delayed. Therefore, prompt and accurate diagnosis is essential for ameliorating the prognosis of patients.

Currently used methods of diagnosing anaplasmosis include detecting anaplasmosis by isolating A. *phagocytophilum* from the blood of a patient and culturing the same, detecting morulae at an early stage in peripheral blood samples through Wright-Giemsa staining using peripheral blood, performing detection in patient sera using an antibody to A. *phagocytophilum,* and the like.

However, methods of diagnosing disease by culturing *A*. *phagocytophilum* require long culture time of several weeks or more, and thus are unsuitable for clinical diagnosis in practice. Indirect immunofluorescence antibody and immunoenzyme methods, which are serological tests, have disadvantages of causing false-positive reactions upon cross-reactivity with other pathogens, exhibiting low sensitivity when used for early disease detection because it takes several days for antibodies to form after the onset of symptoms, and requiring follow-up examination for definitive diagnosis.

In addition, when a very low cut-off values of IgM 1:16 or higher and IgG 1:80 or higher are used as diagnostic criteria for anaplasmosis in a clinical study targeting anaplasmosis patients, IgM sensitivity is only 23% and IgG sensitivity is only 15%, indicating that the current diagnostic method has problems of low speed and low accuracy.

Furthermore, in order to diagnose anaplasmosis using PCR, the 16S rRNA, ankA, and groEL target protein genes were used as diagnostic biomarkers. When these genes are employed as diagnostic markers, it is inconvenient since nested PCR and real-time PCR should be used instead of conventional PCR. Because A. *phagocytophilum* is an intracellular bacterium, the detection sensitivity of conventional PCR is low. Although conducting nested PCR twice improve the sensitivity, this is cumbersome because it consumes more labor and time than necessary and entails increased potential for contamination. Therefore, it is critical to develop a novel diagnostic method that is capable of exhibiting excellent sensitivity without being performed repeatedly.

Accordingly, the present inventors identified P₄₄, which is a multi-copy gene that can be used as a novel biomarker for quick and accurate diagnosis of anaplasmosis with high sensitivity, and devised a primer set capable of detecting anaplasmosis and a kit including the same, thereby completing the present invention.

### SUMMARY OF THE INVENTION

Therefore, the present invention has been made in view of the above problems, and it is one object of the present invention to provide a composition for anaplasmosis diagnostic markers containing a P₄₄ gene.

Other objects of the present invention include providing a diagnostic composition for anaplasmosis containing a substance for measuring the level of the P₄₄ gene or the P₄₄ protein. A primer set or probe designed to identify *Anaplasma phagocytophilum.* A kit for diagnosing anaplasmosis containing the present invention's diagnostic composition for anaplasmosisand a method for providing information to diagnose *Anaplasma phagocytophilum infection* using the present invention's diagnostic kit for anaplasmosis.

In accordance with the present invention, the above and other objects can be accomplished by the provision of a composition as a diagnostic marker for anaplasmosis containing a P₄₄ gene.

In one embodiment of the present invention, the P₄₄ gene may have the nucleotide sequence of SEQ ID NO: 1.

In accordance with another aspect of the present invention, provided is a diagnostic composition for anaplasmosis containing a substance for measuring the level of a P₄₄ gene or P₄₄ protein.

In one embodiment of the present invention, the substance for measuring the level of the P₄₄ gene may be a primer or probe that specifically binds to the P₄₄ gene or P₄₄ mRNA.

In one embodiment of the present invention, the primer may be a primer set selected from the group consisting of a primer set consisting of primers having sequences of SEQ ID NOS: 3 and 4, a primer set consisting of primers having sequences of SEQ ID NOS: 5 and 6, a primer set consisting of primers having sequences of SEQ ID NOS: 7 and 8, a primer set consisting of primers having sequences of SEQ ID NOS: 9 and 10, a primer set consisting of primers having sequences of SEQ ID NOS: 9 and 11, a primer set consisting of primers having sequences of SEQ ID NOS: 12 and 13, and a primer set consisting of primers having sequences of SEQ ID NOS: 14 and 15, and the probe may be selected from the group consisting of probes having sequences of SEQ ID NOS: 16 to 27.

In one embodiment of the present invention, the substance for measuring the level of the P₄₄ protein may be an antibody that specifically recognizes the P₄₄ protein.

In accordance with another aspect of the present invention, provided is a primer set for detecting *Anaplasma phagocytophilum,* wherein the primer set is selected from the group consisting of: a primer set consisting of primers having sequences of SEQ ID NOS: 3 and 4, a primer set consisting of primers having sequences of SEQ ID NOS: 5 and 6, a primer set consisting of primers having sequences of SEQ ID NOS: 7 and 8, a primer set consisting of primers having sequences of SEQ ID NOS: 9 and 10, a primer set consisting of primers having sequences of SEQ ID NOS: 9 and 11, a primer set consisting of primers having sequences of SEQ ID NOS: 12 and 13, and a primer set consisting of primers having sequences of SEQ ID NOS: 14 and 15.

In accordance with another aspect of the present invention, provided is a probe for detecting *Anaplasma phagocytophilum,* wherein the probe is selected from the group consisting of probes having sequences of SEQ ID NOS: 16 to 27.

In accordance with another aspect of the present invention, provided is a diagnostic kit for anaplasmosis containing the diagnostic composition for anaplasmosis of the present invention.

In one embodiment of the present invention, the diagnostic kit may include at least one selected from the group consisting of: a primer set consisting of primers having sequences of SEQ ID NOS: 3 and 4, a primer set consisting of primers having sequences of SEQ ID NOS: 5 and 6, a primer set consisting of primers having sequences of SEQ ID NOS: 7 and 8, a primer set consisting of primers having sequences of SEQ ID NOS: 9 and 10, a primer set consisting of primers having sequences of SEQ ID NOS: 9 and 11, a primer set consisting of primers having sequences of SEQ ID NOS: 12 and 13, a primer set consisting of primers having sequences of SEQ ID NOS: 14 and 15, and a probe selected from the group consisting of probes having sequences of SEQ ID NOS: 16 to 27.

In accordance with another aspect of the present invention, provided is a method for providing information to diagnose infection with anaplasmosis using the diagnostic kit for anaplasmosis containing the diagnostic composition of the present invention.

In one embodiment of the present invention, the diagnostic kit for anaplasmosis may perform a polymerase chain reaction (PCR) using any one primer set selected from the group consisting of: a primer set consisting of primers having sequences of SEQ ID NOS: 3 and 4, a primer set consisting of primers having sequences of SEQ ID NOS: 5 and 6, a primer set consisting of primers having sequences of SEQ ID NOS: 7 and 8, a primer set consisting of primers having sequences of SEQ ID NOS: 9 and 10, a primer set consisting of primers having sequences of SEQ ID NOS: 9 and 11, a primer set consisting of primers having sequences of SEQ ID NOS: 12 and 13, and a primer set consisting of primers having sequences of SEQ ID NOS: 14 and 15, or any one probe selected from the group consisting of probes having sequences of SEQ ID NOS: 16 to 27.

In one embodiment of the present invention, the diagnosis is performed using a PCR method selected from the group consisting of conventional polymerase chain reaction (C-PCR: conventional PCR), nested polymerase chain reaction (N-PCR: nested PCR), multiple polymerase chain reaction, real-time polymerase chain reaction, real-time quantitative polymerase chain reaction, and loop-mediated isothermal amplification (LAMP).

### BRIEF DESCRIPTION OF THE DRAWINGS

The aforementioned and other objects, features and other advantages of the present invention will provide a clearly understanding from the following detailed description taken in conjunction with the accompanying drawings, in which:

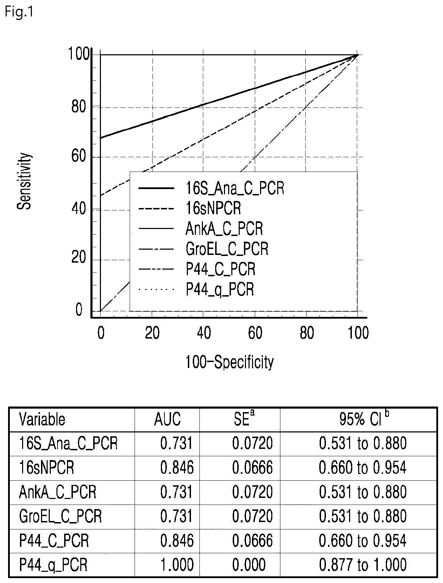

FIG. 1 illustrates the result of a comparative analysis (ROC curve) on the sensitivity of anaplasmosis diagnosis using various PCR methods employing P44, a novel marker for diagnosing anaplasmosis according to the present invention, and conventional markers.

### DETAILED DESCRIPTION OF THE INVENTION

The present invention is characterized by identifying the fact that P₄₄ can be used as a novel biomarker that can accurately and quickly diagnose anaplasmosis at an early stage.

In particular, when searching a method to enhance the speed and accuracy of anaplasmosis diagnosis, the present inventors identified that P₄₄, a multi-copy gene present in *A. phagocytophilum,* can be used as a novel biomarker for diagnosing anaplasmosis.

In particular, the novel biomarker targeted by the present invention is a multi-copy gene. The reason for this is that the multi-copy gene exists as a repeating sequence in a gene cluster, allowing multiple copies to exist in one cell, resulting in extremely high sensitivity. Therefore, a biomarker capable of detecting *A*. *phagocytophilum* was screened from multi-copy genes.

It was found that, among them, P₄₄ is capable of specifically detecting A. *phagocytophilum* with higher sensitivity than other polyclonal genes when used as a marker.

Accordingly, the present invention provides a composition as a diagnostic marker for anaplasmosis containing a P₄₄ gene.

Anaplasmosis (human granulocytic anaplasmosis, HGA) is also known as zoonosis, and infects humans, dogs, cattle, sheep, goats, horses and wild animals, and the causative pathogen thereof is a bacterium called "*Anaplasma phagocytophilum*" that is obligately parasitic within cells.

Therefore, "anaplasmosis" in the present invention refers to a disease caused by infection with *Anaplasma phagocytophilum.*

As used herein, the term "diagnosis" refers to determine a subject's susceptibility to a certain disease or disorder, determine whether or not a subject currently has a specific disease or disorder, or determine the prognosis of a subject with a specific disease or disorder, or therametrics (e.g., monitoring the condition of a subject to provide information about therapeutic efficacy).

As used herein, the term "marker" refers to a substance that can be used to determine whether or not a subject is infected with *Anaplasma phagocytophilum,* or a substance that can be used to distinguish a subject with anaplasmosis caused by infection with *Anaplasma phagocytophilum* from a normal subject, and includes organic biomolecules such as polypeptides or nucleic acids (e.g., mRNA), lipids, glycolipids, glycoproteins, sugars (monosaccharides, disaccharides, oligosaccharides, and the like). For the purposes of the present invention, the diagnostic marker for anaplasmosis may be a P44 gene or a protein expressed by the gene.

Preferably, the P₄₄ gene of the present invention may include the nucleotide sequence of SEQ ID NO: 1, and the P₄₄ protein expressed by the gene may include the amino acid sequence of SEQ ID NO: 2.

In addition, the present invention provides a diagnostic composition for anaplasmosis containing a substance for measuring the level of the P₄₄ gene or P₄₄ protein.

That is, the diagnostic composition can detect *Anaplasma phagocytophilum,* the causative pathogen of anaplasmosis, and the detection may be performed using polymerase chain reaction (PCR).

The substance for measuring the level of the P₄₄ gene may be a primer or probe that specifically binds to the P₄₄ gene or P₄₄ mRNA, and preferably, the primer is selected from the group consisting of: a primer set consisting of primers having sequences of SEQ ID NOS: 3 and 4, a primer set consisting of primers having sequences of SEQ ID NOS: 5 and 6, a primer set consisting of primers having sequences of SEQ ID NOS: 7 and 8, a primer set consisting of primers having sequences of SEQ ID NOS: 9 and 10, a primer set consisting of primers having sequences of SEQ ID NOS: 9 and 11, a primer set consisting of primers having sequences of SEQ ID NOS: 12 and 13, and a primer set consisting of primers having sequences of SEQ ID NOS: 14 and 15, and the probe may be selected from the group consisting of probes having sequences of SEQ ID NOS: 16 to 27.

In addition, the substance for measuring the level of the P₄₄ protein may be an antibody that specifically recognizes the P₄₄ protein.

The diagnostic composition of the present invention may contain at least one component, for example, a buffer solution for reaction, dNTP, Mg²⁺ ions, and DNA polymerase, that may be used for polymerase chain reaction (PCR), in addition to the respective primer sets or probe described above.

The buffer solution for reaction may be 1 to 10 mM Tris HCl or 10 to 40 mM KCl (pH 9.0), and the dNTP may comprise at least one of dATP, dTTP, dGTP, and dCTP.

The diagnostic composition may include a stabilizer and/or a non-reactive dye to improve experimental convenience, stability, and reactivity.

The non-reactive dye should be selected from substances that do not affect the polymerase chain reaction, and is intended to be used for analysis or identification using the polymerase chain reaction product. Substances that satisfy these requirements may be water-soluble dyes such as rhodamine, TAMRA, sodium hypochlorite (household lax), bromophenol blue, xylene cyanol, bromocresol red, and cresol red.

The diagnostic composition may be provided in a liquid form, and is preferably provided in a dried state to increase stability, convenience of storage, and long-term storage. The drying may be performed by a known drying method such as general room-temperature drying, heat drying, freeze drying, or vacuum drying, but any drying method may be used as long as it does not cause loss of components of the composition. The drying method described above may vary depending on the type and amount of the enzyme that is used.

The diagnostic composition is produced and utilized in a stable manner by mixing in a single reaction tube, followed by freezing or drying, thus requiring no separate mixing steps during the polymerase chain reaction. Therefore, errors due to mixing during the reaction can be prevented and stability, reactivity, and storability can be improved.

A commercially available product that contains ingredients other than the primer set or probe, namely, buffer solution for reaction, dNTP, Mg²⁺ ion, and DNA polymerase, may be utilized as the diagnostic composition.

The present invention further includes a primer set or probe that is capable of specifically detecting *Anaplasma phagocytophilum.*

The primer or probe according to the present invention may be a primer or probe that specifically binds to P₄₄ identified in the present invention in order to rapidly and accurately detect *Anaplasma phagocytophilum,* and the primer is preferably selected from the group consisting of: a primer set consisting of primers having sequences of SEQ ID NOS: 3 and 4, a primer set consisting of primers having sequences of SEQ ID NOS: 5 and 6, a primer set consisting of primers having sequences of SEQ ID NOS: 7 and 8, a primer set consisting of primers having sequences of SEQ ID NOS: 9 and 10, a primer set consisting of primers having sequences of SEQ ID NOS: 9 and 11, a primer set consisting of primers having sequences of SEQ ID NOS: 12 and 13, and a primer set consisting of primers having sequences of SEQ ID NOS: 14 and 15.

In addition, preferably, the probe may be selected from the group including SEQ ID NOS: 16 to 27.

In particular, the primer or probe designed in the present invention aims to enable rapid and accurate detection of *Anaplasma phagocytophilum* at an early stage, and can specifically bind to and/or amplify the multi-copy gene P₄₄, and is useful for polymerase chain reaction (PCR).

Furthermore, the present invention provides a diagnostic kit for detecting *Anaplasma phagocytophilum.*

The diagnostic kit contains the diagnostic composition for anaplasmosis of the present invention as described above.

Using the diagnostic kit, the present invention also provides a method of providing information for diagnosing whether or not a subject is infected with *Anaplasma phagocytophilum*

Specifically, the present invention provides a method of providing information to diagnose infection with anaplasmosis, the method including mixing a DNA-containing sample isolated from a biological sample obtained from a subject suspected of having contracted anaplasmosis with the diagnostic composition of the present invention as described above, performing a reaction to amplify the reaction mixture, and analyzing the resultant amplification product.

The diagnostic composition includes the above-described primer set or probe prepared in the present invention, and the reaction to amplify the reaction mixture may be performed by a polymerase chain reaction (PCR) method.

In addition, the analysis method for diagnosis is selected from the group that includes but not limited to conventional polymerase chain reaction (C-PCR: conventional PCR), nested polymerase chain reaction (N-PCR: nested PCR), multiple polymerase chain reaction, real-time polymerase chain reaction, real-time quantitative polymerase chain reaction, and loop-mediated isothermal amplification (LAMP), but is not limited thereto.

The subject suspected of having anaplasmosis is one who have been infected or suspected of having being infected with *Anaplasma phagocytophilum,* and may be selected from domestic animals such as ticks, rats, wild animals, cattle, pigs, sheep, goats, deer, horses, companion animals such as dogs and cats, and humans.

The biological sample may be a body fluid or secretion of the subject and examples thereof include, but are not limited to, blood, serum, plasma, lymph, cerebrospinal fluid, tissue fluid, or the like, or urine, tears, saliva, milk, vomit, feces, and the like, which are secretions from the body, and the biological sample may preferably be blood.

Hereinafter, the present invention will be described in more detail with reference to examples. The examples are provided only for illustration of the present invention, and should not be construed as limiting the scope of the present invention.

### <Example 1>

### Production of primers and probes for detecting and diagnosing anaplasmosis

While performing research to discover a novel biomarker enabling detection of anaplasmosis quickly, accurately, and with high sensitivity, the present inventors visited the Chosun University Hospital. They chose P₄₄ as a target gene that can be used as a biomarker for anaplasmosis diagnosis from among multiple-copy genes that exhibit differences in expression from normal subjects in blood obtained from patients diagnosed with anaplasmosis and infected with the A. *phagocytophilum* strain, and produced primers and probes capable of specifically amplifying P₄₄ as shown in Table 1, and in particular, designed the primers and probes of the present invention shown in Table 1 so as to diagnose anaplasmosis with high sensitivity through only simple PCR.

**[Table 1]**

| Assay Name | Primer, Probes | Base Sequence | Position | Length | Tm | SEQ ID NO. | Product |
|---|---|---|---|---|---|---|---|
| Ana P44 | Sense Primer | GGATGGAAAGAGTGTAAAG | 90 | 19 | 59.1 | 3 | 154 |
| Ana P44 | Anti-sense Primer | CTCGTAACCAATCTCAAG | 243 | 18 | 58.5 | 4 | |
| Ana P44 | Anti-sense Probe | CACCACCAATACCATAACCAACACTG | 214 | 26 | 69 | 16 | |
| Ana P44 | Sense Primer | GGATGGAAAGAGTGTAAAG | 90 | 19 | 59.1 | 3 | 154 |
| Ana P44 | Anti-sense Primer | CTCGTAACCAATCTCAAG | 243 | 18 | 58.5 | 4 | |
| Ana P44 | Anti-sense Probe | TACCATAACCAACACTGCCTTCCATA | 205 | 26 | 69 | 17 | |
| Ana P44 | Sense Primer | GGATGGAAAGAGTGTAAAG | 90 | 19 | 59.1 | 3 | 154 |
| Ana P44 | Anti-sense Primer | CTCGTAACCAATCTCAAG | 243 | 18 | 58.5 | 4 | |
| Ana P44 | Anti-sense Probe | CAATACCATAACCAACACTGCCTTCC | 208 | 26 | 69.1 | 18 | |
| Ana P44 | Sense Primer | GGATGGAAAGAGTGTAAAG | 90 | 19 | 59.1 | 3 | 154 |
| Ana P44 | Anti-sense Primer | CTCGTAACCAATCTCAAG | 243 | 18 | 58.5 | 4 | |
| Ana P44 | Anti-sense Probe | CCAATACCATAACCAACACTGCCTTC | 209 | 26 | 69.1 | 19 | |
| Ana P44 | Sense Primer | GGATGGAAAGAGTGTAAAG | 90 | 19 | 59.1 | 3 | 154 |
| Ana P44 | Anti-sense Primer | CTCGTAACCAATCTCAAG | 243 | 18 | 58.5 | 4 | |
| Ana P44 | Anti-sense Probe | ATACCATAACCAACACTGCCTTCCATA | 206 | 27 | 69.1 | 20 | |
| Ana P44 | Sense Primer | GGATGGAAAGAGTGTAAAG | 90 | 19 | 59.1 | 3 | 154 |
| Ana P44 | Anti-sense Primer | CTCGTAACCAATCTCAAG | 243 | 18 | 58.5 | 4 | |
| Ana P44 | Anti-sense Probe | TACCATAACCAACACTGCCTTCCA | 205 | 24 | 69.2 | 21 | |
| Ana P44 | Sense Primer | GCTATGGAAGGCAGTGTTGG | 178 | 20 | 55.98 | 5 | 73 |
| Ana P44 | Anti-sense Primer | TGAAGCGCTCGTAACCAATC | 231 | 20 | 55.77 | 6 | |
| Ana P44 | Anti-sense Probe | AGCTCAACCCTGGCACCACCA | 207 | 21 | 63.17 | 22 | |
| Ana P44 | Sense Primer | ACAGTCCAGCGTTTAGCAAG | 8 | 20 | 55.59 | 7 | 190 |
| Ana P44 | Anti-sense Primer | CCAACACTGCCTTCCATAGC | 178 | 20 | 55.98 | 8 | |
| Ana P44 | sense Probe | TGACTGGAACACTCCTGATCCTCGGA | 126 | 26 | 62.7 | 23 | |
| Ana P44 | Sense Primer | CCTGATCCTCGGATTGGGTT | 139 | 20 | 56.16 | 9 | 81 |
| Ana P44 | Anti-sense Primer | CCTGGCACCACCAATACCAT | 200 | 20 | 57.02 | 10 | |
| Ana P44 | Anti-sense Probe | CCAACACTGCCTTCCATAGCTACAAGC | 171 | 27 | 62.02 | 24 | |
| Ana P44 | Sense Primer | CCTGATCCTCGGATTGGGTT | 139 | 20 | 56.16 | 9 | 117 |
| Ana P44 | Anti-sense Primer | GGTCTTGAAGCGCTCGTAAC | 236 | 20 | 56.45 | 11 | |
| Ana P44 | Anti-sense Probe | AGCTCAACCCTGGCACCACCA | 207 | 21 | 63.17 | 25 | |
| Ana P44 | Sense Primer | TATTGGTGGTGCCAGGGTT | 204 | 19 | 55.97 | 12 | 156 |
| Ana P44 | Anti-sense Primer | AGGTTATCAGTCTGCCCAGT | 340 | 20 | 55.02 | 13 | |
| Ana P44 | Anti-sense Probe | ACCCTTGGTCTTGAAGCGCTCGT | 239 | 23 | 63.22 | 26 | |
| Ana P44 | Sense Primer | ACAAGTTTGACTGGAACACTCC | 119 | 22 | 55.47 | 14 | 101 |
| Ana P44 | Anti-sense Primer | CCTGGCACCACCAATACCAT | 200 | 20 | 57.02 | 15 | |
| Ana P44 | Anti-sense Probe | CCAACACTGCCTTCCATAGCTACAAGC | 171 | 27 | 62.02 | 27 | |

### <Example 2>

### Detection sensitivity analysis of the present invention using primers and probe for detecting anaplasmosis

A test was performed to determine whether or not the primer and probe for detecting anaplasmosis of the present invention devised in Example 1 can quickly detect anaplasmosis with high sensitivity.

For this purpose, first, among patients who visited Chosun University Hospital from 2016 to 2017, blood from 15 patients diagnosed with anaplasmosis and blood from 15 patients diagnosed with a disease other than anaplasmosis were selected for testing.

Anaplasmosis was defined as a case in which the amount of an antibody against A. *phagocytophilum* increased more than fourfold in immunofluorescence antibody assay (IFA), and the positive control used herein was blood collected from 15 confirmed patients. In addition, the negative control used herein was blood obtained from 15 patients in whom an antibody was not detected by immunofluorescence antibody assay (IFA) and who were diagnosed with diseases other than anaplasmosis. Each of the blood samples obtained above was centrifuged, a buffy coat was collected therefrom, genomic genes were extracted therefrom, and PCR was performed using the primers or probes of the present invention shown in Table 1 to analyze the diagnostic potential and sensitivity of anaplasmosis. In addition, regarding comparative groups, PCR analysis was performed on 16S rRNA (GenBank: CP000235), ankA (GenBank: AF020521) and groEL-STG (GenBank: CP000235) genes. These genes are known to be conventional anaplasmosis diagnostic markers as template DNA. Using specific primers to compare the detection sensitivity of P₄₄, a novel biomarker identified in the present invention, as well as the primers and probes for detecting the same.

**[Table 2]**

| PCR method | Groel C-PCR | | AnkA PCR | | | 16S PCR | | MSP2 C-PCR | | P44 C-PCR | | 16S N-PCR | | P44 Q-PCR | |
|---|---|---|---|---|---|---|---|---|---|---|---|---|---|---|---|
| | Case | Control | Case | | Control | Case | Control | Case | Control | Case | Control | Case | Control | Case | Control |
| Test positive | 6 | 0 | | 6 | 0 | 6 | 0 | 6 | 0 | 11 | 0 | 11 | 0 | 15 | 0 |
| Test negative | 9 | 15 | | 9 | 15 | 8 | 15 | 9 | 15 | 4 | 15 | 4 | 15 | 0 | 15 |
| Total | 15 | 15 | | 15 | 15 | 14 | 15 | 15 | 15 | 15 | 15 | 15 | 15 | 15 | 15 |
| Sensitivity, %(95% CI) | 40(17-67) | | 40(17-67) | | | 43(19-70) | | 43(19-70) | | 73(45-91) | | 73(45-91) | | 100(75-100) | |
| Specificity, %(95% CI) | 100(75-100) | | 100(74-100) | | | 100(74-100) | | 100(75-100) | | 100(75-100) | | 100(75-100) | | 100(75-100) | |
| PPV, % (95% CI) | 100(52-100) | | 100(52-100) | | | 100(52-100) | | 100(52-100) | | 100(68-100) | | 100(68-100) | | 100(75-100) | |
| NPV, % (95% CI) | 63(41-80) | | | 63(41-80) | | 65(43-83) | | 65(43-83) | | 79(54-93) | | 79(54-93) | | 100(75-100) | |
| Time | 3-4h | | 3-4h | | | 3-4h | | 3-4h | | 3-4h | | 6-7h | | 1-2h | |

In addition, the respective primer sequences used for the test are shown in Table 3 below, and PCR was respectively performed under the conditions described in Tables 4 and 5 below.

**[Table 3]**

| PCR | Primer | Primer Name | Primer Sequence | SEQ ID NO. | Product size (bp) |
|---|---|---|---|---|---|
| groEL N-PCR 1^{st} PCR | 1^{st} Forward | GRO607F | GAAGATGCWGTWGGWTGTACKGC | 28 | 688 |
| | 1^{st} Reverse | GRO1294R | AGMGCTTCWCCTTCWACRTCYTC | 29 | |
| groEL N-PCR 2^{nd} PCR/C-PCR | 2nd Forward | GRO677F | ATTACTCAGAGTGCTTCTCARTG | 30 | 445 |
| | 2nd Reverse | GRO1121R | TGCATACCRTCAGTYTTTTCAAC | 31 | |
| ankA N-PCR 1^{st} PCR | 1^{st} Forward | ANK-F1 | GAAGAAATTACAACTCCTGAAG | 32 | 705 |
| | 1^{st} Reverse | ANK-R1 | CAGCCAGATGCAGTAACGTG | 33 | |
| ankA N-PCR 2^{nd} PCR/C-PCR | 2nd Forward | ANK-F2 | TTGACCGCTGAAGCACTAAC | 34 | 664 |
| | 2nd Reverse | ANK-R2 | ACCATTTGCTTCTTGAGGAG | 35 | |
| 16s N-PCR 1^{st} PCR | 1^{st} Forward | AE1-F | AAGCTTAACACATGCAAGTCGAA | 36 | 1406 |
| | 1^{st} Reverse | AE1-R | AGTCACTGA CCCAACCTTAAATG | 37 | |
| 16s N-PCR 2^{nd} PCR/C-PCR | 2nd Forward | EE-3 | GTCGAACGGATTATTCTTTATAGCTTGC | 38 | 926 |
| | 2nd Reverse | EE-4 | CCCTTCCGTTAAGAAGGATCTAATCTCC | 39 | |
| msp2(P44) C-PCR | Forward | msp2F | ATGTCCATGGCTATAGTCATGGCTG | 40 | 430 |
| | Reverse | msp2R | ACCTCGAGTTAAGCTAACTCCTTAGCT | 41 | |
| msp2 N-PCR 1^{st} PCR | 1^{st} Forward | Anapmsp21F | TTATGATTAGGCCTTTGGGCATG | 42 | 1079 |
| | 1^{st} Reverse | Anapmsp21R | TCAGAAAGATACACGTGCGCCC | 43 | |
| msp2 N-PCR 2^{nd} PCR | 2nd Forward | Anapmsp22F | GGTTACATAAGGGCCGCAAAGGTG | 44 | 467 |
| | 2nd Reverse | Anapmsp22R | CCGGCGCATGTGTAAGGTGAAA | 45 | |
| P44 Q-PCR | Forward | anaP44 90F | GGATGGAAAGAGTGTAAAG | 46 | |
| | Reverse | anaP44 243R | CTCGTAACCAATCTCAAG | 47 | 153 |
| | Probe | anaP44 anti-214P | [TET]CACCACCAATACCATAACCAACACT[BHQ1] | 48 | |

**[Table 4]**

| Step | groEL N-PCR 1stPCR | | groEL N-PCR 2ndPCR/C-PCR | | ankA N-PCR 1stPCR | | ankA N-PCR 2ndPCR/C-PCR | |
|---|---|---|---|---|---|---|---|---|
| | Temperature | Time | Temperature | Time | Temperature | Time | Temperature | Time |
| 1 | 95 | 5m | 95 | 5m | 95 | 5m | 95 | 5m |
| 2 | 95 | 30s | 95 | 30s | 95 | 30s | 95 | 30s |
| 3 | 54 | 30s | 50 | 30s | 53 | 30s | 52 | 30s |
| 4 | 72 | 90s | 72 | 60s | 72 | 60s | 72 | 60s |
| 5 | Step2 | 35 cycle | Step2 | 5 cycle | Step2 | 35 cycle | Step2 | 5 cycle |
| 6 | 72 | 5m | 95 | 30s | 72 | 5m | 95 | 5m |
| 7 | | | 53 | 30s | | | 54 | 30s |
| 8 | | | 72 | 60s | | | 72 | 30s |
| 9 | | | Step6 | 5 cycle | | | Step6 | 60s |
| 10 | | | 95 | 30s | | | 72 | 5m |
| 11 | | | 57 | 30s | | | | |
| 12 | | | 72 | 60s | | | | |
| 13 | | | Step10 | 25 cycle | | | | |
| 14 | | | 72 | 5m | | | | |

**[Table 5]**

| Step | 16s N-PCR 1^{st} PCR | | 16s N-PCR 2^{nd} PCR/C-PCR | | msp2(P44) C-PCR | |
|---|---|---|---|---|---|---|
| | Temperature | Time | Temperature | Time | Temperature | Time |
| 1 | 95 | 5m | 95 | 5m | 95 | 5m |
| 2 | 95 | 30s | 95 | 30s | 95 | 30s |
| 3 | 59 | 30s | 5b | 30s | 56 | 30s |
| 4 | 72 | 90s | 72 | 60s | 72 | 60s |
| 5 | Step2 | 35 cycle | Step2 | 30 cycle | Step2 | 35 cycle |
| 6 | 72 | 5m | 72 | 5m | 72 | 5m |

| Step | msp2 N-PCR 1^{st} PCR | | msp2 N-PCR 2^{nd} PCR | | P44 Q-PCR | |
|---|---|---|---|---|---|---|
| | Temperature | Time | Temperature | Time | Temperature | Time |
| 1 | 95 | 5m | 95 | 5m | 95 | 5m |
| 2 | 95 | 30s | 95 | 30s | 95 | 5s |
| 3 | 54 | 30s | 57 | 30s | 51 | 5s |
| 4 | 72 | 60s | 72 | 60s | Scan | TET |
| 5 | Step2 | 35 cycle: | Step2 | 35 cycle | Step2 | 45 cycle |
| 6 | 72 | 5m | 72 | 5m | 25 | 1m |

As shown in Table 2 and FIG. 1, the analysis revealed that PCR amplification products were not observed in any of the 15 negative control group samples, whereas PCR amplification products were detected in the blood of patients diagnosed with anaplasmosis. However, it took 3 to 7 hours to obtain detection results for target genes (16S rRNA, ankA, and groEL-STG) used as comparative groups, whereas it took 1 to 2 hours to detect P44, which was a novel biomarker, using the primer of the present invention.

In addition, the diagnostic sensitivity of anaplasmosis, obtained through detection of P₄₄, was 100% when the primer devised in the present invention was used, indicating that sensitivity and accuracy are very high. In contrast, it was found that the diagnostic sensitivity of conventionally used target genes (16S rRNA, ankA, groEL-STG) was low.

In addition, it was found that when C-PCR was performed using the P₄₄ gene of the present invention, the analysis time was considerably shorter than when N-PCR was performed using other target genes. When real-time PCR (Q-PCR) was performed on P₄₄ gene, 100% sensitivity was obtained, which has much higher sensitivity and specificity than markers and detection primers that have been developed and used to date, indicating that the P₄₄ gene may accurately detect and diagnose anaplasmosis.

As previously stated, P₄₄, which is a novel biomarker for diagnosing anaplasmosis according to the present invention, is a multicopy gene that exists in a large number of copies in the *Anaplasma phagocytophilum* genome, allowing detection of *Anaplasma phagocytophilum* infection with high sensitivity using only a small amount of DNA compared to conventional diagnostic marker genes. In addition, the primer set or probe for detecting and amplifying P₄₄ according to the present invention is capable of quickly and simply detecting anaplasmosis with high specificity and sensitivity, and is thus useful for early diagnosis of anaplasmosis.

Although the preferred embodiments of the present invention have been disclosed, those skilled in the art will appreciate that various modifications, additions and substitutions are possible, without departing from the scope and spirit of the invention as disclosed in the accompanying claims. Therefore, the disclosed embodiments should be considered from an illustrative point of view rather than a limiting point of view. The scope of the present invention is defined by the claims rather than the aforementioned description, and all differences falling within the scope of equivalents thereto should be construed as falling within the scope of the present invention.

## Claims

1. A composition as a diagnostic marker for anaplasmosis comprising a P₄₄ gene.

2. The composition according to claim 1, wherein the P₄₄ gene comprises a nucleotide sequence of SEQ ID NO: 1.

3. A diagnostic composition for anaplasmosis comprising a substance for measuring a level of a P₄₄ gene or P₄₄ protein.

4. The diagnostic composition according to claim 3, wherein the substance for measuring the level of the P₄₄ gene is a primer or probe that specifically binds to the P₄₄ gene or P₄₄ mRNA.

5. The diagnostic composition according to claim 4, wherein the primer is selected from the group consisting of:
a primer set consisting of primers having sequences of SEQ ID NOS: 3 and 4;
a primer set consisting of primers having sequences of SEQ ID NOS: 5 and 6;
a primer set consisting of primers having sequences of SEQ ID NOS: 7 and 8;
a primer set consisting of primers having sequences of SEQ ID NOS: 9 and 10;
a primer set consisting of primers having sequences of SEQ ID NOS: 9 and 11;
a primer set consisting of primers having sequences of SEQ ID NOS: 12 and 13; and
a primer set consisting of primers having sequences of SEQ ID NOS: 14 and 15, and
the probe is selected from the group consisting of probes having sequences of SEQ ID NOS: 16 to 27.

6. The diagnostic composition according to claim 3, wherein the substance for measuring the level of the P₄₄ protein is an antibody that specifically recognizes the P₄₄ protein.

7. A primer set for detecting *Anaplasma phagocytophilum,*
wherein the primer set is selected from the group consisting of:
a primer set consisting of primers having sequences of SEQ ID NOS: 3 and 4;
a primer set consisting of primers having sequences of SEQ ID NOS: 5 and 6;
a primer set consisting of primers having sequences of SEQ ID NOS: 7 and 8;
a primer set consisting of primers having sequences of SEQ ID NOS: 9 and 10;
a primer set consisting of primers having sequences of SEQ ID NOS: 9 and 11;
a primer set consisting of primers having sequences of SEQ ID NOS: 12 and 13; and
a primer set consisting of primers having sequences of SEQ ID NOS: 14 and 15.

8. A probe for detecting *Anaplasma phagocytophilum,*
wherein the probe is selected from the group consisting of probes having sequences of SEQ ID NOS: 16 to 27.

9. A diagnostic kit for anaplasmosis comprising the diagnostic composition according to claim 3.

10. The diagnostic kit according to claim 9, wherein the diagnostic kit comprises at least one selected from the group consisting of:
a primer set consisting of primers having sequences of SEQ ID NOS: 3 and 4;
a primer set consisting of primers having sequences of SEQ ID NOS: 5 and 6;
a primer set consisting of primers having sequences of SEQ ID NOS: 7 and 8;
a primer set consisting of primers having sequences of SEQ ID NOS: 9 and 10;
a primer set consisting of primers having sequences of SEQ ID NOS: 9 and 11;
a primer set consisting of primers having sequences of SEQ ID NOS: 12 and 13;
a primer set consisting of primers having sequences of SEQ ID NOS: 14 and 15; and
a probe consisting of probes having sequences of SEQ ID NOS: 16 to 27.

11. A method for providing information to diagnose infection with anaplasmosis using the diagnostic kit for anaplasmosis comprising the diagnostic composition according to claim 3.

12. The method according to claim 11, comprising performing polymerase chain reaction (PCR) using any one primer set selected from the group consisting of:
a primer set consisting of primers having sequences of SEQ ID NOS: 3 and 4;
a primer set consisting of primers having sequences of SEQ ID NOS: 5 and 6;
a primer set consisting of primers having sequences of SEQ ID NOS: 7 and 8;
a primer set consisting of primers having sequences of SEQ ID NOS: 9 and 10;
a primer set consisting of primers having sequences of SEQ ID NOS: 9 and 11;
a primer set consisting of primers having sequences of SEQ ID NOS: 12 and 13; and
a primer set consisting of primers having sequences of SEQ ID NOS: 14 and 15, or
any one probe selected from the group consisting of probes having sequences of SEQ ID NOS: 16 to 27.

13. The method according to claim 11, wherein the diagnosis is performed using a PCR method selected from the group consisting of conventional polymerase chain reaction (C-PCR: conventional PCR), nested polymerase chain reaction (N-PCR: nested PCR), multiple polymerase chain reaction, real-time polymerase chain reaction, real-time quantitative polymerase chain reaction, and loop-mediated isothermal amplification (LAMP).


<110>    Industry Academic Cooperation Foundation of Chosun University

<120>    Use of P44 as diagnostic marker of anaplasmosis

<130>    NPDC77794

<160>    48

<170>    KoPatentIn 3.0

<210>    1
<211>    1242
<212>    DNA
<213>    Artificial Sequence

<220>
<223>    P44 gene sequence


<400>    1
atgttctcat tttctttaaa aggcagacct tcctttttcg aaatagcctt ttctttagga        60

agcgtaatga tgtctatggc tatagtcatg gctgggagtg atgtcagggc tcatgatgac       120

gttagcgctt tggagactgg tggtgcggga tatttctatg ttggtttgga ttacagtcca       180

gcgtttagca agataagaga ttttagtata agggagagta acggagagac taaggcagta       240

tatccatact taaaggatgg aaagagtgta aagctagagt cacacaagtt tgactggaac       300

acacctgatc ctcggattgg gtttaaggac aacatgcttg tagctatgga aggcagtgtt       360

gggtacggta ttggtggtgc cagggttgag cttgagattg gttacgagcg tttcaagact       420

aagggtatta gagatagtgg tagtaaggaa gatgaagctg atacagtata tctactagct       480

aaggagttag cttatgatgt tgttactggg cagactgata accttgctgc tgctcttgcc       540

aaaacctccg gtaaggacat cgttcagttt gctaaggcgg tggagatctc ttaccctagt       600

atcgatggga aggtttgtag tggaaagcat gcggcgcttg cagcaaacac gaacgcggag       660

aaaaagtacg cggttgagcc tgcgaacggc ggaacagacg ggagcacgtc gcagtgtagt       720

ggtttgagta atggtagtgc ggaggctgct cataaatatt tgagtaagtt tgtaagtctt       780

acgggagtgg ttgaagggaa aaactggcct actggtagat cgagtaacaa cagcaatagc       840

attgtagttg gagcacctaa tagtaacgca aacgcgatgg ccaaagatct agtaaaggaa       900

ctaacaccag aggagaaaac catagttgcc gggttactag ctaaaactat tgaagggggt       960

gaggttgtag aaattagggc ggtttcttct acttctgtaa tggtcaatgc ttgttatgat      1020

cttcttagtg aaggtttagg tgttgttcct tatgcttgtg ttggtttagg tggtaacttc      1080

gtaggtgttg ttgatgggca tatcactcct aagcttgctt ataggttaaa ggctggcttg      1140

agttatcagc tctctcctga aatctccgct tttgcgggtg gattctacca tcgtgttgtg      1200

ggagatggcg tttatgatga tctgccggag cctatgcaat aa                         1242

<210>    2
<211>    80
<212>    PRT
<213>    Artificial Sequence

<220>
<223>    P44 aminoacid sequence


<400>    2
Met Phe Ser Phe Ser Leu Lys Gly Arg Pro Ser Phe Phe Glu Ile Ala
1               5                   10                  15

Phe Ser Leu Gly Ser Val Met Met Ser Met Ala Ile Val Met Ala Gly
            20                  25                  30

Ser Asp Val Arg Ala His Asp Asp Val Ser Ala Leu Glu Thr Gly Gly
        35                  40                  45

Ala Gly Tyr Phe Tyr Val Gly Leu Asp Tyr Ser Pro Ala Phe Ser Lys
    50                  55                  60

Ile Arg Asp Phe Ser Ile Arg Glu Ser Asn Gly Glu Thr Lys Ala Val
65                  70                  75                  80


<210>    3
<211>    19
<212>    DNA
<213>    Artificial Sequence

<220>
<223>    Ana P44 Sense Primer


<400>    3
ggatggaaag agtgtaaag                                                19


<210>    4
<211>    18
<212>    DNA
<213>    Artificial Sequence

<220>
<223>    Ana P44 Antisense Primer


<400>    4
ctcgtaacca atctcaag                                                 18


<210>    5
<211>    20
<212>    DNA
<213>    Artificial Sequence

<220>
<223>    Ana P44 Sense Primer


<400>    5
gctatggaag gcagtgttgg                                               20

<210> 6
<211> 20
<212> DNA
<213> Artificial Sequence

<220>
<223> Ana P44 Antisense Primer


<400> 6
tgaagcgctc gtaaccaatc 20


<210> 7
<211> 20
<212> DNA
<213> Artificial Sequence

<220>
<223> Ana P44 Sense Primer


<400> 7
acagtccagc gtttagcaag 20


<210> 8
<211> 20
<212> DNA
<213> Artificial Sequence

<220>
<223> Ana P44 Antisense Primer


<400> 8
ccaacactgc cttccatagc 20


<210> 9
<211> 20
<212> DNA
<213> Artificial Sequence

<220>
<223> Ana P44 Sense Primer


<400> 9
cctgatcctc ggattgggtt 20


<210> 10
<211> 20
<212> DNA
<213> Artificial Sequence

<220>
<223> Ana P44 Antisense Primer

<400>    10
cctggcacca ccaataccat                                                    20


<210>    11
<211>    20
<212>    DNA
<213>    Artificial Sequence

<220>
<223>    Ana P44 Antisense Primer


<400>    11
ggtcttgaag cgctcgtaac                                                    20


<210>    12
<211>    19
<212>    DNA
<213>    Artificial Sequence

<220>
<223>    Ana P44 Sense Primer


<400>    12
tattggtggt gccagggtt                                                     19


<210>    13
<211>    20
<212>    DNA
<213>    Artificial Sequence

<220>
<223>    Ana P44 Antisense Primer


<400>    13
aggttatcag tctgcccagt                                                    20


<210>    14
<211>    22
<212>    DNA
<213>    Artificial Sequence

<220>
<223>    Ana P44 Sense Primer


<400>    14
acaagtttga ctggaacact cc                                                 22


<210>    15
<211>    20
<212>    DNA
<213>    Artificial Sequence

<220>
<223>    Ana P44 Antisense Primer

<400>    15
cctggcacca ccaataccat                                                    20


<210>    16
<211>    26
<212>    DNA
<213>    Artificial Sequence

<220>
<223>    Ana P44 Antisense Probe


<400>    16
caccaccaat accataacca acactg                                             26


<210>    17
<211>    26
<212>    DNA
<213>    Artificial Sequence

<220>
<223>    Ana P44 Antisense Probe


<400>    17
taccataacc aacactgcct tccata                                             26


<210>    18
<211>    26
<212>    DNA
<213>    Artificial Sequence

<220>
<223>    Ana P44 Antisense Probe


<400>    18
caataccata accaacactg ccttcc                                             26


<210>    19
<211>    26
<212>    DNA
<213>    Artificial Sequence

<220>
<223>    Ana P44 Antisense Probe


<400>    19
ccaataccat aaccaacact gccttc                                             26


<210>    20
<211>    27
<212>    DNA
<213>    Artificial Sequence

<220>
<223>    Ana P44 Antisense Probe


<400>    20
ataccataac caacactgcc ttccata                                          27


<210>    21
<211>    24
<212>    DNA
<213>    Artificial Sequence

<220>
<223>    Ana P44 Antisense Probe


<400>    21
taccataacc aacactgcct tcca                                             24


<210>    22
<211>    21
<212>    DNA
<213>    Artificial Sequence

<220>
<223>    Ana P44 Antisense Probe


<400>    22
agctcaaccc tggcaccacc a                                                21


<210>    23
<211>    26
<212>    DNA
<213>    Artificial Sequence

<220>
<223>    Ana P44 Sense Probe


<400>    23
tgactggaac actcctgatc ctcgga                                           26


<210>    24
<211>    27
<212>    DNA
<213>    Artificial Sequence

<220>
<223>    Ana P44 Antisense Probe


<400>    24
ccaacactgc cttccatagc tacaagc                                          27


<210>    25
<211>    21
<212>    DNA

<213>    Artificial Sequence

<220>
<223>    Ana P44 Antisense Probe


<400>    25
agctcaaccc tggcaccacc a                                                21


<210>    26
<211>    23
<212>    DNA
<213>    Artificial Sequence

<220>
<223>    Ana P44 Antisense Probe


<400>    26
acccttggtc ttgaagcgct cgt                                              23


<210>    27
<211>    27
<212>    DNA
<213>    Artificial Sequence

<220>
<223>    Ana P44 Antisense Probe


<400>    27
ccaacactgc cttccatagc tacaagc                                          27


<210>    28
<211>    23
<212>    DNA
<213>    Artificial Sequence

<220>
<223>    GRO607F F Primer


<400>    28
gaagatgcwg twggwtgtac kgc                                              23


<210>    29
<211>    23
<212>    DNA
<213>    Artificial Sequence

<220>
<223>    GRO1294R R Primer


<400>    29
agmgcttcwc cttcwacrtc ytc                                              23


<210>    30

<211>    23
<212>    DNA
<213>    Artificial Sequence

<220>
<223>    GRO677F F Primer


<400>    30
attactcaga gtgcttctca rtg                                              23


<210>    31
<211>    23
<212>    DNA
<213>    Artificial Sequence

<220>
<223>    GRO1121R R Primer


<400>    31
tgcataccrt cagtyttttc aac                                              23


<210>    32
<211>    22
<212>    DNA
<213>    Artificial Sequence

<220>
<223>    ANK-F1 F Primer


<400>    32
gaagaaatta caactcctga ag                                               22


<210>    33
<211>    20
<212>    DNA
<213>    Artificial Sequence

<220>
<223>    ANK-R1 R Primer


<400>    33
cagccagatg cagtaacgtg                                                  20


<210>    34
<211>    20
<212>    DNA
<213>    Artificial Sequence

<220>
<223>    ANK-F2 F Primer


<400>    34
ttgaccgctg aagcactaac                                                  20

<210>    35
<211>    20
<212>    DNA
<213>    Artificial Sequence

<220>
<223>    ANK-R2 R Primer


<400>    35
accatttgct tcttgaggag                                                    20


<210>    36
<211>    23
<212>    DNA
<213>    Artificial Sequence

<220>
<223>    AE1-F F Primer


<400>    36
aagcttaaca catgcaagtc gaa                                                23


<210>    37
<211>    23
<212>    DNA
<213>    Artificial Sequence

<220>
<223>    AE1-R R Primer


<400>    37
agtcactgac ccaaccttaa atg                                                23


<210>    38
<211>    28
<212>    DNA
<213>    Artificial Sequence

<220>
<223>    EE-3 F Primer


<400>    38
gtcgaacgga ttattcttta tagcttgc                                           28


<210>    39
<211>    28
<212>    DNA
<213>    Artificial Sequence

<220>
<223>    EE-4 R Primer


<400>    39

cccttccgtt aagaaggatc taatctcc 28

<210> 40
<211> 25
<212> DNA
<213> Artificial Sequence

<220>
<223> msp2F F Primer

<400> 40
atgtccatgg ctatagtcat ggctg 25

<210> 41
<211> 27
<212> DNA
<213> Artificial Sequence

<220>
<223> msp2R R Primer

<400> 41
acctcgagtt aagctaactc cttagct 27

<210> 42
<211> 23
<212> DNA
<213> Artificial Sequence

<220>
<223> Anapmsp21F F Primer

<400> 42
ttatgattag gcctttgggc atg 23

<210> 43
<211> 22
<212> DNA
<213> Artificial Sequence

<220>
<223> Anapmsp21R R Primer

<400> 43
tcagaaagat acacgtgcgc cc 22

<210> 44
<211> 24
<212> DNA
<213> Artificial Sequence

<220>
<223> Anapmsp22F F Primer

<400>    44
ggttacataa gggccgcaaa ggtg                                              24


<210>    45
<211>    22
<212>    DNA
<213>    Artificial Sequence

<220>
<223>    Anapmsp22R R Primer


<400>    45
ccggcgcatg tgtaaggtga aa                                                22


<210>    46
<211>    19
<212>    DNA
<213>    Artificial Sequence

<220>
<223>    anaP44 90F F Primer


<400>    46
ggatggaaag agtgtaaag                                                    19


<210>    47
<211>    18
<212>    DNA
<213>    Artificial Sequence

<220>
<223>    anaP44 243R R Primer


<400>    47
ctcgtaacca atctcaag                                                     18


<210>    48
<211>    25
<212>    DNA
<213>    Artificial Sequence

<220>
<223>    anaP44 anti-214P Probe


<400>    48
caccaccaat accataacca acact                                             25